Europäisches Patentamt

(19) European Patent Office

Office européen des brevets

(11) Publication number: **0 098 136**
A2

# EUROPEAN PATENT APPLICATION

(21) Application number: 83303694.0

(22) Date of filing: 27.06.83

(51) Int. Cl.$^3$: **C 12 N 9/04**
C 12 P 7/24, C 12 P 7/26
C 12 P 7/40

(30) Priority: 28.06.82 US 392608

(43) Date of publication of application:
11.01.84 Bulletin 84/2

(84) Designated Contracting States:
DE FR GB

(71) Applicant: Exxon Research and Engineering Company
P.O.Box 390 180 Park Avenue
Florham Park New Jersey 07932(US)

(72) Inventor: Hou, Ching-Tsang
14 Pendleton Place
Edison New Jersey(US)

(72) Inventor: Laskin, Allen I.
RD 3/Box 392T
Somerset New Jersey(US)

(72) Inventor: Patel, Ramesh Narsibhai
15 Mockingbird Road
Edison New Jersey(US)

(74) Representative: Field, Roger Norton et al,
ESSO Engineering (Europe) Ltd. Patents & Licences
Apex Tower High Street
New Malden Surrey KT3 4DJ(GB)

(54) Primary and secondary alcohol dehydrogenase enzymes and the use thereof.

(57) A newly discovered and isolated heat-stable NAD(P)$^+$-linked secondary alcohol-specific dehydrogenase enzyme is derived from microorganisms grown under aerobic conditions in a culture medium containing a carbon-containing compound having at least two carbon atoms, preferably a $C_2$-$C_{10}$ alkane or a $C_2$-$C_{10}$ alkyl radical donating compound, as the major carbon and energy source. The purified form of this enzyme, in the presence of NAD(P)$^+$, is capable of converting primary and secondary alcohols, diols and aldehydes to their corresponding oxidation products and acetone to propanol.

In addition, a newly discovered NAD(P)$^+$-linked primary alcohol dehydrogenase enzyme can be isolated from the same microorganism source.

EFFECT OF pH ON ENZYME ACTIVITY

FIG. I

0098136

- 1 -

The present invention relates to a newly discovered and isolated heat-stable enzyme which is a nicotinamide adenine dinucleotide $(NAD^+)$-linked secondary alcohol-specific dehydrogenase enzyme derived from microorganisms grown aerobically in the presence of a carbon-containing compound having at least two carbon atoms, preferably a $C_2$-$C_{10}$ alkyl compound such as, e.g., a $C_2$-$C_{10}$ alkane or alcohol as the major carbon and energy source. The purified form of the enzyme herein may be used, in the presence of $NAD^+$ or $NADP^+$, to produce methyl ketones and aldehydes from secondary and primary alcohols, respectively, and to oxidize diols and aldehydes, and for other industrial synthetic and analytical uses. The invention also relates to a newly discovered $NAD^+$-linked alcohol dehydrogenase enzyme preferential to primary alcohols.

$NAD^+$-dependent primary alcohol dehydrogenase from liver and baker's yeast preferentially oxidizes primary alcohols and, at a lower rate (about 10% of the ethanol activity), secondary alcohols. see generally C-I. Bränden et al., _The Enzymes_, Boyer, P.D., ed., vol. 11 (Academic Press: New York, 1975) pp. 103-109 and also F.M. Dickinson et al., _Biochem J._, _104_, 165 (1967) and F.M. Dickinson et al., _Nature_ (Lond.), _214_, 31 (1967). NAD(P)-dependent primary alcohol dehydrogenase enzymes were also isolated from _Pseudomonas_, _Escherichia coli_, _Leuconostoc mesenteroides_ and _Rhizopus javanicus_.

In U.S. Pat. Nos. 4,241,184 and 4,250,259 a novel $NAD^+$-linked secondary alcohol-specific alcohol dehydrogenase is disclosed which was derived from methylotrophic microbes. Such an enzyme is important in producing methyl ketones.

This invention relates to a primary or secondary alcohol dehydrogenase enzyme characterized as being derived from cells of a microorganism or genetically engineered derivative thereof or natural mutant thereof which has been previously grown under aerobic conditions in a nutrient medium containing a carbon-containing compound having at least two carbon atoms which provides the carbon and energy source for growth of the cells of the microorganism and induces alcohol dehydrogenase enzyme activity in the microorganism, derivative or mutant. One of the enzymes identified preferentially oxidizes primary alcohols to the corresponding oxidation products under aerobic conditions in the presence of $NAD^+$ or $NADP^+$ and has the properties of a primary alcohol dehydrogenase. Another of the enzymes identified is a thermally stable secondary alcohol dehydrogenase characterized as converting short-chain primary and secondary alcohols, diols and aldehydes to the corresponding oxidation products in the presence of $NAD^+$ or $NADP^+$. On purification 46 fold the secondary alcohol dehydrogenase enzyme is found to have a molecular weight of about $145,000 \pm 5,000$ dalton as determined by polyacrylamide gel column chromatography and to be stable at a temperature of at least $50^\circ C$, preferably $60-85^\circ C$, for over one hour.

According to the invention herein described, alcohols, diols and aldehydes (preferably $C_1-C_5$ primary alcohols, $C_3-C_7$ secondary alcohols, $C_3-C_5$ diols, and $C_1-C_4$ aldehydes) are microbiologically converted to their corresponding oxidation products by a process comprising contacting such alcohol, diol, or aldehyde in a reaction medium with microbial cells derived from a microorganism, a genetically engineered derivative or natural mutant thereof, or an enzyme preparation prepared from said cells until the oxidation product is produced in at least isolatable amounts, wherein said microbial cells, derivatives or enzyme preparations have been previously heated at a temperature of up to about 90°C and wherein said microorganism has been grown under aerobic conditions in a nutrient medium containing a carbon-containing compound having at least two carbon atoms which provides the carbon and energy source for growth of the cells of the microorganism and induces alcohol dehydrogenase enzyme activity in the microorganism, derivative, mutant or preparation, and producing the product in isolatable amounts.

The thermally stable enzyme is also capable of reducing methyl ketones, preferably acetone, to the corresponding alcohols, but at a lower rate.

Figure 1 represents a plot of enzyme activity versus pH for the purified secondary alcohol dehydrogenase enzyme from _Pseudomonas fluorescens_ NRRL B-1244 used to oxidize 2-propanol.

Figure 2 represents a plot of enzyme activity versus temperature from 10 to 90°C for the purified secondary alcohol dehydrogenase enzyme from _Pseudomonas fluorescens_ NRRL B-1244 used to oxidize 2-propanol.

Figure 3 represents a plot of enzyme activity (% of maximum) versus time for the purified secondary alcohol dehydrogenase enzyme from _Pseudomonas fluorescens_ NRRL B-1244 when heated as a dilute solution at 65°C, 85°C and 92°C, using 2-propanol as substrate.

The term "microorganism" is used herein in its broadest sense to include not only bacteria, but also yeasts, filamentous fungi, actinomycetes and protozoa. Preferably, the microorganisms will include bacteria. The microorganism herein is defined as capable of

utilizing $C_2$ or greater alkyl compounds as the major or sole carbon and energy source.

The expression "genetically engineered derivatives of a microorganism" is used herein to refer to living cells with an altered hereditary apparatus in the sense recognized by those skilled in the art and includes artificial mutants and recombinant DNA-produced microorganisms.

The term "enzyme preparation" is used to refer to any composition of matter that exhibits the desired dehydrogenase enzymatic activity. The term is used to refer, for example, to live whole cells, dried cells, cell-free particulate and soluble fractions, cell extracts, and refined and concentrated preparations derived from the cells, especially purified alcohol dehydrogenases. Enzyme preparations may be either in dry or liquid form. The term also includes the immobilized form of the enzyme, e.g., the whole cells of the microorganisms or enzyme extracts which are immobilized or bound to an insoluble matrix by covalent chemical linkages, absorption and entrapment of the enzyme within a gel lattice having pores sufficiently large to allow the molecules of the substrate and the product to pass freely, but sufficently small to retain the enzyme. The term "enzyme preparation" also includes enzymes retained within hollow fiber membranes, e.g., as disclosed by Rony, Biotechnology and Bioengineering (1971).

The term "secondary alcohol dehydrogenase enzyme" will be used hereinafter to refer to the thermally stable enzyme which preferentially catalyzes oxidation of secondary alcohols but is also capable of oxidizing, to a lesser extent in general, primary alcohols, diols and aldehydes and of reducing ketones to alcohols. Thus, the enzyme herein is not strictly secondary alcohol specific, the term "secondary alcohol dehydrogenase" being used for convenience wherever it occurs and for purposes of distinguishing this enzyme from the other alcohol dehydrogenase enzyme.

The term "primary alcohol dehydrogenase enzyme" will be used hereinafter to refer to the enzyme which has been identified as a catalyst preferential to primary alcohols, thus distinguishing it from the thermally stable alcohol dehydrogenase enzyme, which preferentially oxidizes secondary alcohols.

The term "soluble fraction" refers to the enzyme activity in the soluble extract (supernatant) after centrifuging broken cells at 10,000 to 20,000 x g for 15-30 minutes or greater.

The term "$C_2$-$C_{10}$ alkyl compound," used to describe what is preferably used as the growth substrate, refers to a $C_2$-$C_{10}$ alkane such as, e.g., ethane, propane, butane, isobutane, pentane, 2-methylbutane, hexane, octane, decane, etc., or a $C_2$-$C_{10}$ alkyl radical donating compound, which is a compound which will donate, e.g., ethyl, propyl, butyl, hexyl, decyl, etc. radicals, such as ethanol, propanol, butanol, hexanol, octanol, decanol, ethylamine, propylamine, butyl formate, ethyl carbonate, etc. Such alkyl radical donating compounds can also be characterized as growth substrates which are capable of inducing dehydrogenase enzyme activity in $C_2$ or greater-utilizing microorganisms. Preferably, the $C_2$-$C_{10}$ alkyl compound herein is a $C_2$-$C_4$ alkyl compound, and more preferably $C_2$-$C_4$ n-alkanes, $C_2$-$C_4$ primary alcohols and $C_2$-$C_4$ alkylamines.

The term "short chain alcohols, diols and aldehydes" refers to the preferred substrates for the secondary alcohol dehydrogenase, i.e., $C_1$-$C_5$ primary alcohols, $C_3$-$C_7$ secondary alcohols, $C_3$-$C_5$ diols, and $C_1$-$C_4$ aldehydes.

As one embodiment of the present invention, there is provided an NAD(P)$^+$-linked alcohol dehydrogenase enzyme derived from cells of microorganisms which have been aerobically grown in the presence of a carbon-containing compound having at least two carbon atoms which provides the carbon and energy source for growth of the cells of the microorganism and induces alcohol dehydrogenase enzyme activity in the cells.

Another embodiment herein includes a process for converting primary and secondary alcohols, diols and aldehydes, preferably $C_1$-$C_5$ primary alcohols, $C_3$-$C_7$ secondary alcohols, $C_3$-$C_5$ diols, and $C_1$-$C_4$ aldehydes, to the corresponding oxidation products by contacting the desired substrate with microbial cells or enzyme preparations prepared therefrom which have been previously heated up to a temperature of about 90°C, preferably 30-85°C. The time allowed for heating will vary dramatically with the temperature, but is preferably up to two hours.

The microorganisms themselves are previously grown under aerobic conditions in the presence of a carbon-containing compound having at least two carbon atoms, preferably a $C_2$-$C_{10}$ alkane or a $C_2$-$C_{10}$ alkyl radical donating compound such as ethanol, propanol, butanol, propylamine, propyl formate, butyl formate, ethyl carbonate, propyl carbonate, etc.

The most preferred primary alcohol substrates for this purpose are methanol, ethanol, 1-propanol, 1-butanol and 1-pentanol. The most preferred secondary alcohol substrates herein are 2-propanol, 2-butanol, 2-pentanol, 3-pentanol isobutanol, cyclohexanol and benzyl alcohol. The most preferred diols herein are 1,2-butanediol, 1,3-butanediol, 2,3-butanediol and 1,2-propanediol; and the most preferred aldehydes are formaldehyde, propanal and butanal.

The present invention includes the following features:

The alcohol dehydrogenase enzymes are derived from microorganisms grown on $C_2$ or higher alkyl compounds and can also be found in thermophiles. Alcohol dehydrogenase activity is observed in the cell-free soluble extract of the broken cells of the $C_2$ or greater-grown strains. The cell-free system requires a cofactor, specifically $NAD^+$, for its activity.

On further purification of the crude extract of one strain, Pseudomonas fluorescens NRRL B-1244, two types of enzymes are detected. The primary alcohol dehydrogenase enzyme preferentially oxidizes primary alcohols and exhibits properties similar to those of well-known primary alcohol dehydrogenase enzymes, e.g., the alcohol:$NAD^+$ oxidoreductase from bakers' yeast and the alcohol dehydrogenase as described by Bränden et al., "The Enzymes", pp. 104 ff, supra.

The secondary alcohol dehydrogenase enzyme, upon purification 46 fold, is found to oxidize stoichiometrically secondary alcohols to methyl ketones, primary alcohols to aldehydes, and diols and aldehydes to their corresponding oxidized products in the presence of $NAD(P)^+$, the best oxidation occurring with secondary alcohols, followed by diols, primary alcohols and aldehydes. The highest activity is observed for 2-propanol, 2-butanol and 1,3-butanediol. The enzyme shows no preference when oxidizing optically active isomers. The purified enzyme will also reduce acetone to propanol in the presence of $NADH_2$.

The purified secondary alcohol dehydrogenase enzyme has a molecular weight of about 145,000 ± 5,000 dalton as determined by polyacrylamide gel column chromatography.

The purified secondary alcohol dehydrogenase enzyme has an optimum activity at pH 7-9 and at a temperature of 60-70°C. The activation energy for the enzyme is 8.2 kcal.

The purified secondary alcohol dehydrogenase enzyme is stable when heated up to 85°C for over one hour but is deactivated at 92°C and is denatured on boiling.

The purified secondary alcohol dehydrogenase activity is inhibited by strong thio-reagents but not by most metal chelating agents and not by up to 400 mM 2-butanol in the oxidation mixture.

The microorganisms which may be employed in the present invention can be defined as any which have the capacity for aerobic growth in a nutrient medium containing a carbon-containing compound having at least two carbon atoms. Examples of suitable organisms for purposes herein are given below.

Several newly discovered and isolated microorganism strains useful herein have been identified according to the classification system described in Bergey's Manual of Determinative Bacteriology, Robert S. Breed et al., eds., 8th ed. (Baltimore: Williams and Wilkins Co., 1974) and have the following designations:

TABLE I

| Microorganism Strain Name | ER&E Designation | U.S.D.A. Agriculture Research Center Designation |
|---|---|---|
| 1. Acinetobacter sp. | (CRL 67 P11) | NRRL B-11,313 |
| 2. Actinomyces sp. | (CRL 66 P7) | NRRL 11,314 |
| 3. Arthrobacter sp. | (CRL 60 P1) | NRRL B-11,315 |
| 4. Arthrobacter sp. | (CRL 68 E1) | NRRL B-11,316 |
| 5. Arthrobacter sp. | (CRL 70 B1) | NRRL B-11,317 |
| 6. Brevibacterium sp. | (CRL 52 P3P) | NRRL B-11,318 |
| 7. Brevibacterium sp. | (CRL 56 P6Y) | NRRL B-11,319 |
| 8. Brevibacterium sp | (CRL 61 P2) | NRRL B-11,320 |
| 9. Corynebacterium sp. | (CRL 63 P4) | NRRL B-11,321 |
| 10. Mycobacterium sp. | (CRL 51 P2Y) | NRRL B-11,322 |
| 11. Mycobacterium sp. | (CRL 62 P3) | NRRL B-11,323 |
| 12. Mycobacterium sp. | (CRL 69 E2) | NRRL B-11,324 |
| 13. Nocardia sp. | (CRL 55 P5Y) | NRRL 11,325 |
| 14. Nocardia sp. | (CRL 57 P7Y) | NRRL 11,326 |
| 15. Nocardia sp. | (CRL 64 P5) | NRRL 11,327 |
| 16. Pseudomonas sp. | (CRL 53 P3Y) | NRRL B-11,329 |
| 17. Pseudomonas sp. | (CRL 54 P4Y) | NRRL B-11,330 |
| 18. Pseudomonas sp. | (CRL 58 P9Y) | NRRL B-11,331 |
| 19. Pseudomonas sp. | (CRL 65 P6) | NRRL B-11,332 |
| 20. Pseudomonas sp. | (CRL 71 B2) | NRRL B-11,333 |

Each of the above-designated strains have been deposited at the United States Department of Agriculture, Agriculture Research Service, Northern Regional Research Laboratory (NRRL), Peoria, Illinois 61604 /and have on the 20th June 1978 received from NRRL the individual NRRL designations as indicated above pursuant to a contract between NRRL and the assignee of this patent application, Exxon Research and Engineering Company (ER&E). The contract with NRRL provides for permanent availability of the progeny of these strains to the public upon the issuance of the U.S. patent describing and identifying the deposits or the publication or laying open to the public of any U.S. or foreign patent application or patent corresponding to this application, whichever occurs first. These strains have however now been made available to the public from the 13th June 1983.

The assignee of the present application has agreed that, if any of these strains on deposit should die, or be lost or destroyed when cultivated under suitable conditions, it will be promptly replaced on notification with a viable culture of the same strain. It should be understood, however, that the availability of a deposit does not constitute a licence to practise the subject invention in derogation of patent rights granted by governmental action.

The taxonomical and morphological characteristics of these newly isolated strains are shown below:

MORPHOLOGICAL AND TAXONOMICAL
CHARACTERISTICS OF MICROORGANISM STRAINS

1. _Acinetobacter_ sp. CRL 67 strain P11 (NRRL B-11, 313) Cells are very short and plump in logarithmic growth phase, approaching coccus shape in stationary phase. Cells are predominantly in pairs and short chains. Non-spore-forming, non-motile cells. Grow aerobically on $C_2$ to $C_{10}$ alkanes, $C_2$ to $C_{10}$ primary alcohols, propylamine, and nutrient agar. Do not grow on methane.

2. <u>Actinomyces</u> sp. CRL 66 strain P7 (NRRL 11,314) Organisms are gram-positive, irregular staining cells, non acid-fast, non-spore-forming and non-motile. Many filamentous cells with branching. Produce rough colonies on plate and pink pigmentation. Grow aerobically on $C_2$ to $C_{10}$ alkanes, $C_2$ to $C_{10}$ primary alcohols, propylamine and nutrient agar. Do not grow on methane.

3. <u>Arthrobacter</u> sp. CRL 60 strain P1 (NRRL B-11,315) Culture is composed of coccoid cells. In some old cultures, cells are spherical to ovoid or slightly elongated. Organisms are gram-positive, non acid-fast, strictly aerobic, hydrolyze gelatin and reduce nitrate. Grow aerobically at the expense of $C_2$ to $C_{10}$ alkanes, $C_2$ to $C_{10}$ primary alcohols, propylamine, succinate and nutrient agar. Do not grow on methane.

4. <u>Arthrobacter</u> sp. CRL 68 strain E1 (NRRL B-11,316) Culture is composed of coccoid cells. In old cultures cells are spherical to ovoid or slightly elongated. Organisms are gram-positive, non-acid-fast, strictly aerobic. Hydrolyze gelatin and reduce nitrate. Grow aerobically on $C_2$ to $C_{10}$ alkanes, $C_2$ to $C_{10}$ primary alcohols, ethylamine, propylamine, and nutrient agar. Do not grow on methane.

5. <u>Arthrobacter</u> sp. CRL 70 strain B1 (NRRL B-11,317) Culture is composed of coccoid cells. In some old cultures cells are spherical to ovoid or slightly elongated. Organisms are gram-positive, non-acid-fast, strictly aerobic. Hydrolyze gelatin and reduce nitrate.

Grow aerobically on $C_2$ to $C_{10}$ alkanes, $C_2$ to $C_{10}$ primary alcohols, butylamine, and nutrient agar. Do not grow on methane.

6. Brevibacterium sp. CRL 52 P3P (NRRL B-11,318) Produce yellow, shiny raised colonies on mineral salt agar plates in the presence of $C_2$ to $C_{10}$ alkanes and primary alcohols. Also grow on nutrient media. Aerobic, rod-shaped organisms.

7. Brevibacterium sp. CRL 56 P6Y (NRRL B-11,319) Produce shiny, raised, yellow colonies on mineral salt agar plates in the presence of $C_2$ to $C_{10}$ alkanes and primary alcohols. Also grow on nutrient media. Aerobic, rod- to pear-shaped organisms.

8. Brevibacterium sp. CRL 61 strain P2 (NRRL B-11,320) Cells have coryneform morphology. They have "snapping" mode of cell division. Organisms are aerobic, forming yellowish to orange pigment on plates. Grow aerobically on $C_2$ to $C_{10}$ alkanes, $C_2$ to $C_{10}$ primary alcohols, propylamine, succinate and nutrient agar. Do not grow on methane.

9. Corynebacterium sp. CRL 63 strain P4 (NRRL B-11,321) Cells are straight or curved rods, frequently swollen at one or both ends. Gram-positive, usually stain unevenly and often contain metachromatic granules which stain bluish purple with methylene blue. Organisms liquify gelatin. Grow aerobically on $C_2$ to $C_{10}$ alkanes, $C_2$ to $C_{10}$ primary alcohols, propylamine, succinate and nutrient agar. Do not grow on methane.

0098136

- 13 -

10. <u>Mycobacterium</u> sp. CRL 51 P2Y (NRRL B-11,322) Produce small white colonies on mineral salt agar in the presence of $C_2$ to $C_{10}$ alkanes and primary alcohols. Also grow on nutrient medium. The organisms are non-motile, gram-negative, aerobic rods.

11. <u>Mycobacterium</u> sp. CRL 62 strain P3 (NRRL B-11,323) Cells are short, plump rods, slightly curved with rounded or occasionally thickened ends. Acid-fast organism. Produce smooth, moist, shiny colonies with deep yellow pigmentation. Grow aerobically on $C_2$ to $C_{10}$ alkanes, $C_2$ to $C_{10}$ primary alcohols, pro-pylamine, and nutrient agar. Do not grow on methane. Reduce nitrate and catalase test positive.

12. <u>Mycobacterium</u> sp. CRL 69 Strain E2 (NRRL B-11,324) Cells are short, plump rods, slightly curved with rounded or occasionally thickened rods. Acid-fast organism. Produce smooth, moist, shiny colonies with deep yellow pigmentation. Grow aerobically on $C_2$ to $C_{10}$ alkanes, $C_2$ to $C_{10}$ primary alcohols, ethyl-amine, propylamine and nutrient agar. Do not grow on methane.

13. <u>Nocardia</u> sp. CRL 55 P5Y (NRRL 11,325) Produce slow-growing shiny, yellow colonies on mineral salt agar plates in the presence of $C_2$ to $C_{10}$ alkanes and primary alcohols. Also grow on nutrient media. Gram-positive, rod-shaped, aerobic organisms.

14. <u>Nocardia</u> sp. CRL 57 P7Y (NRRL 11,326) Produce shiny, raised cream- to yellow-colored colonies on mineral salt agar plates in the presence of $C_2$ to $C_{10}$ alkanes and primary alcohols. Also grow on nutrient media. Gram-positive, rod-shaped organisms.

15. Nocardia sp. CRL 64 strain P5 (NRRL 11,327) Colonies are dry and flaky. Cells produce branched mycelial which fragment into irregular bacillary and coccoid cells. Produce yellowish colonies on plates. Grow aerobically on $C_2$ to $C_{10}$ alkanes, $C_2$ to $C_{10}$ primary alcohols, propylamine, succinate and nutrient agar. Do not grow on methane.

16. Pseudomonas sp. CRL 53 P3Y (NRRL B-11,329) Produce small yellow colonies on mineral salt agar plates in the presence of $C_2$ to $C_{10}$ alkanes and primary alcohols. Also grow on nutrient media. Gram-negative, aerobic, motile, rod-shaped organisms.

17. Pseudomonas sp. CRL 54 P4Y (NRRL B-11,330) Produce shiny yellow colonies on mineral salt agar plates in the presence of $C_2$ to $C_{10}$ alkanes and primary alcohols. Also grow on nutrient media. Gram-negative, motile, aerobic rods.

18. Pseudomonas sp. CRL 58 P9Y (NRRL B-11,331) Produce yellow colonies on mineral salt agar plates in the presence of $C_2$ to $C_{10}$ alkanes and primary alcohols. Also grow on nutrient media. Gram-negative, motile, aerobic rods.

19. Pseudomonas sp. CRL 65 strain P6 (NRRL B-11,332) Organisms are gram-negative, aerobic rods. Motile by polar, monotrichous flagella. Do not produce flourescent pigment. Nitrate is denitrified by these organisms. Grow aerobically on $C_2$ to $C_{10}$ alkanes, $C_2$ to $C_{10}$ primary alcohols, propylamine and nutrient agar. Do not grow on methane.

20. Pseudomonas sp. CRL 71 strain B2 (NRRL B-11,333) Organisms are gram-negative, aerobic rods. Motile by polar monotrichous flagella. Do not produce flourescent pigment. Nitrate is denitrified by organisms. Grow aerobically on $C_2$ to $C_{10}$ alkanes, $C_2$ to $C_{10}$ primary alcohols, butylamine and nutrient agar. Do not grow on methane.

The newly discovered and isolated strains described above were obtained from soil samples from the Bayway Refinery in Linden, New Jersey, and from lake water samples from Warinanco Park, Linden, New Jersey. The samples were screened for the microorganisms by growth under oxygen and propane. The microorganisms were then isolated, purified and maintained by the procedure described below.

The maintenance of the cultures of these newly discovered and isolated strains should be carefully controlled. The preferred means for isolation and maintenance of the cultures is as follows. One gram of the soil or lake water samples is suspended in 10 ml of mineral salt medium as described below in Table II and allowed to settle at room temperature for one hour. The supernatant solution is inoculated into 300 ml flasks containing 50 ml of mineral salt medium. The enrichment flasks are incubated at 30°C on a shaker under an atmosphere of gaseous ethane, propane, or butane and air (1:1, vol./vol.). Within 96 hours the cultures become turbid. Serial dilutions of the enrichment cultures are prepared and spread onto mineral salt agar plates. These plates should be incubated in glass dessicators which have lids with an airtight seal and external sleeves with a tooled hose connection. Dessicators are to be evacuated and filled with a gas mixture of ethane, propane or butane and air (1:1 v/v). Incubation should be at 30°C. Cultures will survive in these dessicators for three months at 4°C. However, frequent transfer of cultures is preferred.

In addition to the new strains mentioned above, other microorganism strains which are useful herein are enumerated below. These are all known strains and are classified according to the classification system described in Bergey's Manual of Determinative Bacteriology, Robert S. Breed et al., eds., 8th ed., (Baltimore: Williams & Wilkins Co., 1974). Subcultures of each strain were deposited either with the depository of the American Type Culture Collection (ATCC) in Rockville, Maryland 20852 or with the depository of the U.S. Department of Agriculture, Northern Regional Research Laboratory (NRRL) in Peoria, Illinois 61604 and are now publicly available. Each subculture received from the depository the individual ATCC or NRRL strain designation as indicated below. It will be noted that some of the microorganisms have two designations as indicated in the ATCC Catalog of Strains I, 15th ed., 1982.

| Culture | ATCC or NRRL Designation |
|---|---|
| 1. Rhodococcus rhodochrous (Arthrobacter sp.) | ATCC 19140 |
| 2. Pseudomonas aeruginosa (Alcaligenes sp.) | ATCC 15525 |
| 3. Arthrobacter petroleophagus | ATCC 21494 |
| 4. Arthrobacter simplex | ATCC 21032 |

| 5. | Pseudomonas aeruginosa | ATCC 15528 |
| | (Brevibacterium insectiphilum) | |
| 6. | Brevibacterium sp. | ATCC 14649 |
| 7. | Brevibacterium fuscum | ATCC 15993 |
| 8. | Alcaligenes eutrophus | ATCC 17697 |
| | (Hydrogenomonas eutropha) | |
| 9. | Mycobacterium album | ATCC 29676 |
| 10. | Mycobacterium paraffinicum | ATCC 12670 |
| 11. | Rhodococcus rhodochrous | ATCC 29670 |
| | (Mycobacterium rhodochrous) | |
| 12. | Rhodococcus rhodochrous | ATCC 29672 |
| | (Mycobacterium rhodochrous) | |
| 13. | Rhodococcus rhodochrous | ATCC 184 |
| | (Mycobacterium rhodochrous) | |
| 14. | Rhodococcus sp. | ATCC 21499 |
| | (Nocardia neoopaca) | |
| 15. | Rhodococcus rhodochrous | |
| | (Nocardia paraffinica) | ATCC 21198 |
| 16. | Pseudomonas crucurae | NRRL B-1021 |
| 17. | Pseudomonas fluorescens | NRRL B-1244 |
| 18. | Pseudomonas cepacia | ATCC 17616 |
| | (Pseudomonas multivorans) | |
| 19. | Pseudomonas putida Type A | ATCC 17453 |
| 20. | Pseudomonas aeruginosa | ATCC 15522 |
| | (Pseudomonas ligustri) | |

The morphological and taxonomical characteristics of the above strains, as well as literature references or patents where the strains are described, are indicated as follows:

Rhodococcus rhodochrous (Arthrobacter sp.) ATCC 19140 (M. Goldberg et al., Can. J. Microbiol., 3, 329 (1957)) The organisms are Gram-negative rods, usually very short and plump in logarithmic phase, approaching coccus shape in stationary phase, predominantly in pairs and short chains, immotile, catalase positive. Grow aerobically on nutrient broth, succinate, glucose, aliphatic hydrocarbons and n-paraffins. Do not grow on methane.

Pseudomonas aeruginosa (Alcaligenes sp.) ATCC 15525 (U.S. Pat. No. 3,308,035, Re. 26,502 and U.S. Pat. No. 3,301,766 to Esso Research and Engineering Co.) The organisms are Gram-negative small rods, immotile. Ferment glucose. Grow aerobically on $C_2-C_{30}$ aliphatic hydrocarbons, e.g., $C_2-C_{30}$ n-paraffins and $C_6-C_{30}$ olefins.

Arthrobacter petroleophagus ATCC 21494 (Strain No. 2-15) (U.S. Pat. No. 3,762,997 to Nippon Oil Co. Ltd.) The organisms are Gram-positive or negative, immotile rods. Grow aerobically on $C_2-C_5$ and $C_{11}-C_{18}$ n-paraffins, glucose, citrate and glycerol. Do not grow on methane.

Arthrobacter simplex ATCC 21032 (B-129 strain) (U.S. Pat. No. 3,622,465 to Allied Chemical Corp.) The organisms are Gram-negative cells in both rod and coccus forms, motile, non-spore forming. Grow aerobically on $C_3-C_{18}$ n-paraffins. Do not grow on methane.

Pseudomonas aeruginosa (Brevibacterium insectiphilum) ATCC 15528 (U.S. Pat. No. 3,308,035 and Re. 26,502 to Esso Research and Engineering Co.) The organisms are Grampositive small rods, immotile. Ferment glucose. Grow aerobically on $C_2-C_{30}$ aliphatic hydrocarbons, e.g., $C_2-C_{30}$ n-paraffins and $C_6-C_{30}$ olefins.

Brevibacterium sp. ATCC 14649 (U.S. Pat. No. 3,239,394 to Merck & Co. Inc.) The organisms are Gram-positive short rods. Grow on $C_2-C_{10}$ alkanes and alcohols and on nutrient agar. Do not grow on methane.

Brevibacterium fuscum ATCC 15993 (N. Saito et al., Agr. Biol. Chem., 28, 48 (1964) and N. Saito, Biochem. J.,

57, 7 (1965)) The organisms are Gram-negative, short, unbranched rods, reproducing by simple cell division and placed in the family Brevibacteriaceae, motile. Produce brownish, yellowish or orange pigments. Grow aerobically on nutrient broth, glucose, succinate, aliphatic hydrocarbons and paraffins. Do not grow on methane.

Alcaligenes eutrophus (Hydrogenomonas eutropha) ATCC 17697 (Intern. J. Syst. Bacteriol., 19, 385 (1969) (type strain), B.F. Johnson et al., J. Bacteriol., 107, 468 (1971) and D.H. Davis et al., Arch. Mikrobiol., 70, 2 (1970)) The organisms are Gram-negative unicellular rods, motile with peritrichous flagella. Non-spore forming. Colonies are opaque, white or cream colored. Grow aerobically on nutrient broth, glucose, succinate, aliphatic hydrocarbons and paraffins. Do not grow on methane.

Mycobacterium album ATCC 29676 (J. J. Perry et al., J. Bacteriol., 112, 513 (1972), J. Gen. Microbiol., 82, 163 (1974)) The organisms are not readily stainable by Gram's method but are usually considered Gram-positive. Immotile, slightly curved or straight rods, sometimes branching, filamentous or mycelin-like growth may occur. Acid-alcohol fast at some stage of growth. Grow aerobically on paraffins and aliphatic hydrocarbons. Do not grown on methane.

Mycobacterium paraffinicum ATCC 12670 (J. B. Davis et al., App. Microbiol., 4, 310 (1956) to Magnolia Petroleum Co.) The organisms are Gram-positive slender rods, immotile and acid-alcohol fast at some stage of growth. Irish lipid content in cells and cell walls. Colonies are regularly or variably yellow or orange usually due to carotinoid pigments. Grow aerobically on paraffinic hydrocarbons such as $C_2$-$C_{10}$ n-alkanes,

aliphatic hydrocarbons, ethanol and acetate. Do not grow on methane.

Rhodococcus rhodochrous (Mycobacterium rhodochrous) ATCC 29670 (J.J. Perry et al., J. Bacteriol., 112, 513 (1972)) The organisms are not readily stainable by Gram's method but usually are considered Gram-positive. Immotile, short, plump rods, slightly curved with rounded or occasionally thickened ends, branching rare but occasionally Y-shaped cells observed. Acid-alcohol fast. Yellow to orange pigments. Grow aerobically on paraffins and aliphatic hydrocarbons. Do not grow on methane.

Rhodococcus rhodochrous (Mycobacterium rhodochrous) ATCC 29672 (J. J. Perry et al., J. Bacteriol., 94, 1919 (1967), J. Bacteriol., 96, 318 (1968), Arch. Mikro., 91, 87 (1973), J. Gen. Microbiol., 82, 163 (1974), and J. Bacteriol., 118, 394 (1974) Same characteristics as ATCC 29670.

Rhodococcus rhodochrous (Mycobacterium rhodochrous) ATCC 184 (R.S. Breed, J. Bacteriol., 73, 15 (1957)) Same characteristics as ATCC 29670.

Rhodococcus sp. (Nocardia neoopaca) ATCC 21499 (Strain 2-53) (U.S. Pat. No. 3,762,997 to Nippon Oil Co. Ltd.) The organisms are Gram-positive rods or filaments, immotile. Grow aerobically on $C_2$-$C_4$ and $C_{11}$-$C_{18}$ n-paraffins, glucose, gluconate, citrate and succinate. Do not grow on methane.

Rhodococcus rhodochrous (Nocardia paraffinica) ATCC 21198 (U.S. Pat. No. 3,751,337 to Kyowa Hakko Kogyo Co. Ltd.) The organisms are Gram-positive rods or cells, immotile. Ferment sugars. Grow aerobically on $C_2$-$C_4$ and $C_{12}$-$C_{17}$ n-alkanes. Do not grow on methane.

Pseudomonas crucurae NRRL B-1021 (Bergey's Manual of Determinative Bacteriology, 8th ed., 1974, supra) The organisms are Gram-negative, aerobic rods. Motile by polar monotrichous flagella. Do not produce fluorescent pigment. Grow aerobically on $C_2$ to $C_{10}$ alkanes, $C_2$ to $C_{10}$ primary alcohols, butylamine, and nutrient agar. Do not grow on methane.

Pseudomonas fluorescens NRRL B-1244 (Bergey's Manual of Determinative Bacteriology, 8th ed., 1974, supra) The organisms are Gram-negative, aerobic rods, motile. Produce fluorescent pigment. Hydrolyze gelatin. Non-spore forming. Produce small yellow colonies on mineral salt plates in the presence of $C_2$ to $C_{10}$ alkanes and primary alcohols. Also grow on nutrient media. Do not grow on methane.

Pseudomonas cepacia (Pseudomonas multivorans) ATCC 17616 (R.Y. Stanier et al., J. Gen. Microbiol., 43, 159 (1966) and R. W. Ballard et al., J. Gen. Microbiol., 60, 199 (1970)) The organisms are Gram-negative, unicellular rods, motile with polar multitrichous flagella. Hydrolyze gelatin. Produce phenazine pigment. Non-spore forming. Do not hydrolyze starch. Grow aerobically on succinate, glucose, nutrient broth, paraffins, and aliphatic hydrocarbons, including $C_2$-$C_4$ alcohols and butylamine. Do not grow on methane.

Pseudomonas putida Type A ATCC 17453 (R. Y. Stanier et al., J. Gen. Microbiol., 43, 159 (1966)) The organisms are Gram-negative unicellular rods, motile with polar multitrichous flagella. Do not hydrolyze gelatin or denitrify. Produce diffusible fluorescent pigment. Non-spore forming. Grow aerobically on succinate, nutrient broth, glucose, paraffins and aliphatic hydrocarbons, including $C_2$-$C_4$ alcohols and butylamine. Do not grow on methane.

_Pseudomonas aeruginosa_ (_Pseudomonas ligustri_) ATCC 15522 (U.S. Pat. No. 3,308,035, Re. 26,502 and U.S. Pat. No. 3,301,766 to Esso Research and Engineering Co.) The organisms are Gram-negative small rods, motile. Ferment starch and glucose. Produce fluorescent and phenazine pigment. Non-spore forming. Grow aerobically on $C_2-C_{30}$ aliphatic hydrocarbons, e.g., $C_2-C_{30}$ n-paraffins and $C_6-C_{30}$ olefins.

It will be understood that genetically engineered derivatives may also be used in producing microbial cells and enzyme preparations derived from these cells.

## TABLE II
### MAINTENANCE OF CULTURES

All of the organisms are preferably subcultured every two weeks on mineral salts agar plates which contain medium having the following composition:

| | |
|---|---|
| $Na_2HPO_4$ | 0.21 g |
| $NaH_2PO_4$ | 0.09 g |
| $NaNO_3$ | 2.0 g |
| $MgSO_4 \cdot 7H_2O$ | 0.2 g |
| KCl | 0.04 g |
| $CaCl_2$ | 0.015 g |
| $FeSO_4 \cdot 7H_2O$ | 1 mg |
| $CuSO_4 \cdot 5H_2O$ | 0.01 mg |
| $H_3BO_4$ | 0.02 mg |
| $MnSO_4 \cdot 5H_2O$ | 0.14 g |
| $ZnSO_4$ | 0.02 mg |
| $MoO_3$ | 0.02 mg |
| Agar | 15 g |
| Water | 1 liter |

In commercial processes for the propagation of microorganisms, it is generally necessary to proceed by stages. These stages may be few or many, depending on the nature of the process and the characteristics of the microorganisms. Ordinarily, propagation is started by inoculating cells from a slant of a culture into a presterilized nutrient medium usually contained in a flask. In the flask, growth of the microorganisms is encouraged by various means, e.g., shaking for thorough aeration, and maintenance of suitable temperature. This step or stage is repeated one or more times in flasks or vessels containing the same or larger volumes of nutrient medium. These stages may be conveniently referred to as culture development stages. The microorganisms with or without accompanying culture medium, from the last development stage, are introduced or inoculated into a large-scale fermentor to produce commercial quantities of the microorganisms or enzymes therefrom.

Reasons for growing the microorganisms in stages are manifold, but are primarily dependent upon the conditions necessary for the growth of the microorganisms and/or the production of enzymes therefrom. These include stability of the microorganisms, proper nutrients, pH, osmotic relationships, degree of aeration, temperature and the maintenance of pure culture conditions during fermentation. For instance, to obtain maximum yields of the microbial cells, the conditions of fermentation in the final stage may have to be changed somewhat from those practised to obtain growth of the microorganisms in the culture development stages. Maintaining the purity of the medium, also, is an extremely important consideration, especially where the fermentation is performed under aerobic conditions as in the case of the microorganisms herein. If the fermentation is initially started in a large fermentor, a relatively long period of time will be needed to achieve an appreciable yield of microorganisms and/or dehydrogenase enzymes therefrom. This, of course, enhances the possibility of contamination of the medium and mutation of the microorganisms.

The culture media used for growing the microorganisms and inducing the dehydrogenase enzyme system will be comprised of inorganic salts of phosphate, sulfates, and nitrates as well as oxygen and a source of $C_2$ or higher compounds. The fermentation will generally be conducted at temperatures ranging from 5° to about 70°C, preferably at temperatures ranging from about 25° to about 50°C. The pH of the culture medium should be controlled at a pH ranging from about 4 to 9, and preferably from about 5.5 to 8.5, and more preferably from 6.0 to 7.5. The fermentation may be conducted at atmospheric pressures, although higher pressures up to about 5 atmospheres and higher may be employed.

Typically, to grow the microorganisms and to induce the alcohol dehydrogenase enzyme activity, the microorganisms are inoculated into the medium which is contacted with a gas mixture containing the $C_2$ or higher alkyl compound and oxygen. For continuous flow culture the microorganisms may be grown in any suitably adapted fermentation vessel, for example, a stirred baffled fermentor or sparged tower fermentor, which is provided either with internal cooling or an external recycle cooling loop. Fresh medium may be continuously pumped into the culture at rates equivalent to 0.02 to 1 culture volume per hour and the culture may be removed at a rate such that the volume of culture remains constant. A gas mixture containing the $C_2$ or higher alkyl compound and oxygen and possibly carbon dioxide or other gases is contacted with the medium preferably by bubbling continuously through a sparger at the base of the vessel. The source of oxygen for the culture may be air, oxygen or oxygen-enriched air. Spent gas may be removed from the head of the vessel. The spent gas may be recycled either through an external loop or internally by means of a gas inducer impeller. The gas flow rate and recycling should be arranged to give maximum growth of microorganism and maximum utilization of the $C_2$ or higher alkyl compound.

When it is desired to obtain the primary and secondary alcohol dehydrogenase enzyme fraction one first breaks the cells, e.g., by sonication, French pressure cell, etc., and then removes the cellular debris, e.g., centrifuges at 10-20,000 x g for about 15-30 minutes. The microbial cells may be harvested from the growth medium by any of the standard techniques commonly used, for example, flocculation, sedimentation, and/or precipitation, followed by centrifugation and/or filtration. The biomass may also be dried, e.g., by freeze or spray drying and may be used in this form for further use in the alcohol dehydrogenase conversion process. In the case of the primary and secondary alcohol dehydrogenase enzyme system one may conveniently use the enzyme in the form of the soluble extract (which may be optionally immobilized onto an inert carrier).

To put the invention to practice, the primary and secondary alcohol dehydrogenase enzymes are obtained, such as, for example, in the manner described above wherein the microbial cells are derived from the $C_2$ or greater alkyl compound-utilizing microorganisms which have been aerobically grown in a nutrient medium containing the inducing growth substrate or enzyme preparations derived therefrom. The nutrient medium in which the microorganisms are induced and grown may be the culture medium described by Foster and Davis, J. Bacteriol., 91, 1924 (1966). Once the microorganisms have been induced and grown, the microbial cells are preferably harvested, and the resulting resting microbial cells or the enzyme preparation, e.g., resulting from centrifugation may then be used as such to convert the alcohols, diols and aldehydes to the corresponding oxidized products (or methyl ketones to alcohols) in a buffered solution. The mixture of the substrate material and induced microbial cells or enzyme preparation in the buffered solution is incubated until the desired degree of conversion has been obtained. Thereafter, the product is recovered by conventional means, e.g., distillation, etc. The conversion of substrate to the corresponding product is generally conducted at temperatures ranging from about 5 to 90°C at a pH for the reaction medium of about 4 to 9, preferably at temperatures of 50 to 80°C at a reaction medium pH of about 5.5 to 8.5.

The process of the invention may be carried out batchwise, semicontinuously, continuously, concurrently or countercurrently. Optionally, the suspension containing the enzyme preparation or cells of microorganisms and buffer solution is passed downwardly with vigorous stirring countercurrently to an air stream rising in a tube reactor. The top layer is removed from the down-flowing suspension, while culture and remaining buffer solution constituents are recycled, at least partly, with more oxidative substrate and addition of fresh enzyme preparation or induced microbial cell system, as required.

The growth of the microorganisms and the oxidation process may be conveniently coupled by conducting them simultaneously, but separately and using much higher aeration in the oxidation process (e.g., an air excess of at least twice that required for growth, preferably at least five times as much aeration). Both the growth process and the dehydrogenating process may be conducted in the same reactor in sequential or simultaneous operations by alternate use of normal and strong aeration.

The invention is illustrated further by the following examples which, however, are not to be taken as limiting in any respect. All parts and percentages, unless expressly stated otherwise, are by weight.

EXAMPLE 1

Oxidation Using Crude Extracts:

A nutrient medium as described by Foster and Davis, J. Bacteriol., 91, 1924 (1966), supra, having the following composition per liter of water was prepared:

| | |
|---|---|
| $Na_2HPO_4$ | 0.21 g |
| $NaH_2PO_4$ | 0.09 g |
| $NaNO_3$ | 2.0 g |
| $MgSO_4 \cdot 7H_2O$ | 0.2 g |
| $KCl$ | 0.04 g |
| $CaCl_2$ | 0.015 g |
| $FeSO_4 \cdot 7H_2O$ | 1 mg |
| $CuSO_4 \cdot 5H_2O$ | 0.01 mg |
| $H_3BO_4$ | 0.02 mg |
| $MnSO_4 \cdot 5H_2O$ | 0.02 mg |
| $ZnSO_4$ | 0.14 mg |
| $MoO_3$ | 0.02 mg |

The pH of the nutrient medium was adjusted to 7.0 by the addition of acid or base, and 700 ml samples of the nutrient medium were charged into a plurality of 2.5 liter shake flasks. The shake flasks were inoculated with an inoculating loop of cells from an agar plate containing homogeneous colonies of the microorganisms on the plate (the purity of the isolates was confirmed by microscopic examination). The isolates had been maintained on agar plates under an atmosphere of propane and air having a 1:1 v/v gas ratio which had been transferred every two weeks. For growth on 1-propanol and 2-propanol, the medium was supplemented with 0.3% liquid growth substrate. For growth on gaseous alkanes, the gaseous phase of the inoculated flasks was replaced with a gas mixture comprised of propane or methane (control) and air having a ratio of 1:1 on a v/v basis. The inoculated flasks were sealed airtight and were incubated on a rotary shaker at 250 rpm and at 30°C for two days until turbidity in the medium had developed.

The cells thus grown were washed twice with 25 mM potassium phosphate buffer pH 7.0 and suspended in the same buffer solution containing 5mM $MgCl_2$ and deoxyribonuclease (0.05 mg/ml). Cell suspensions at 4°C were disintegrated by a single passage through a French pressure cell at 60 mPa. Disintegrated cell suspensions were centrifuged at 15,000 x g for 15 min. to remove unbroken cells. The cell-free crude extract (enzyme preparation) was used further for the oxidation studies.

The rate of dehydrogenation of the primary and secondary alcohols tested was measured with a fluorescence spectrophotometer by following the formation of reduced $NAD^+$ (excitation at 340 nm, emission at 460 nm). The assay system (3cc) contained 150 μmole sodium phosphate buffer pH 7.0, 1.0 μmole $NAD^+$, a given amount of enzyme preparation, and 20 μmole alcohol substrate. The reaction was initiated by the addition of the substrate alcohol. The product aldehydes or ketones were identified by retention time comparisons and cochromatography with authentic standards using flame ionization gas chromatography (a stainless steel column was maintained isothermally at 110°C). Specific oxidation rates were expressed as nmoles of product formed per min. per mg of protein. The results obtained are shown in Table III.

TABLE III

Oxidation of Primary and Secondary Alcohols by
Cell-Free Crude Extracts of Various Microorganisms

| Microorganism Strain Identification | Growth Substrate | Oxidation Rate (nmoles/min./mg protein) | | | | |
|---|---|---|---|---|---|---|
| | | ethanol | 1-propanol | 2-propanol | 1-butanol | 2-butanol |
| Arthrobacter sp. (CRL 60 P1) NRRL B-11,315 | propane | 6 | 12 | 49 | 18 | 73 |
| Mycobacterium sp. (CRL 51 P2Y) NRRL B-11,322 | propane | 7 | 9.5 | 93 | 14 | 143 |
| Rhodococcus rhodochrous (Nocardia paraffinica) ATCC 21198 | propane | 9 | 9 | 13.5 | 9 | 27 |
| Pseudomonas fluorescens NRRL B-1244 | propane 1-propanol 2-propanol | 5 15 12 | 16 11 15 | 31 20 28 | 9 8 12 | 56 30 48 |
| Pseudomonas cepacia (Pseudomonas multivorans) ATCC 17616 | propane | 2 | 4 | 15 | 1 | 24 |
| Brevibacterium sp. ATCC 14649 | propane | 10 | 19 | 23 | 9 | 42 |
| Methylosinus sporium No. 5 (control) | methane | 0 | 0 | 0 | 0 | 1.2 |

- 30 -

0098136

EXAMPLE 2

Purification of Enzymes:

Purification was conducted in four steps, each carried out at 4°C. Unless otherwise stated, the buffer solution was 0.05 M sodium phosphate buffer pH 7.0 containing 5mM dithiothreitol. The centrifugations were carried out at 10,000 x g for 20 minutes unless otherwise indicated.

Step 1 (crude extract):
Frozen cells of the microorganism Pseudomonas fluorescens NRRL B-1244 (600 g wet weight) were thawed and suspended in one liter of buffer solution. Cell suspensions were disintegrated by two passages through a French pressure cell at 60 mPa. The disintegrated cell suspension was centrifuged to remove cell debris. Into the crude extract obtained, glycerol was added to a final concentration of 5%.

Step 2 (Fractionation with ammonium sulfate):
To the crude extract obtained in step 1, ammonium sulfate was added to 30% saturation. The precipitate formed was centrifuged and discarded. Additional ammonium sulfate was added to the supernatant to 60% saturation. The pH of the solution was continuously adjusted to 7.0 with ammonium hydroxide solution. The precipitate formed was removed by centrifugation and dissolved in a small amount of buffer solution. This fraction containing both primary and secondary alcohol dehydrogenase enzyme activity was dialyzed overnight against 0.005 M sodium phosphate buffer pH 7.0 containing 5 mM dithiothreitol and 5% glycerol.

Step 3 (DEAE-cellulose column chromatography):
The dialyzed fraction prepared in step 2 was applied to a DEAE-cellulose column which had been equilibrated with a 0.005 M sodium phosphate buffer pH 7.0 containing 5mM dithiothreitol and 5% glycerol. The column was washed with a 600 ml 0.05 M phosphate buffer pH 7.0 containing 5mM dithiothreitol and 5% glycerol, and then was eluted by a linear gradient of 0-0.3M NaCl in the same buffer.

Alcohol dehydrogenase activity was separated into two fractions, one eluted at about 0.25M NaCl concentration having activity preference for primary alcohols (primary alcohol dehydrogenase) and another eluted at 0.5M NaCl concentration having activity preference for secondary alcohols (secondary alcohol dehydrogenase). Both alcohol dehydrogenase activity fractions were combined separately. The protein was precipitated by adding ammonium sulfate to give 60% saturation. The precipitate was dissolved in a small volume of buffer solution and was dialyzed against the buffer solution.

The properties of the primary alcohol dehydrogenase resembled those of well-known alcohol dehydrogenases as described by C. Branden et al., "The Enzymes", Boyer, P.D., ed., Vol. 11 (Academic Press: New York, 1975) pp. 103-109, supra.

Step 4 (Polyacrylamide gel column chromatography):
The secondary alcohol dehydrogenase fraction from step 3 was subjected to further purification. A 2-ml sample of the dialyzed active fraction from step 3 was applied to a polyacrylamide gel (Bio-Gel A-1.5m from Bio-Rad Corp.) column (25 mm diameter x 100 cm long) which had been equilibrated with the buffer solution. Each 2 ml fraction was collected. Secondary alcohol dehydrogenase activity was eluted from tube numbers 135 to 150 with a peak at tube 142. The secondary alcohol dehydrogenase activity fractions were combined. Glycerol was added to a final concentration of 5%. The solution was then concentrated in an ultrafiltration unit equipped with a membrane.

Step 5 (Agarose affinity chromatography):

The concentrated solution from step 4 was applied to an agarose (Affi-Gel Blue) affinity column (0.8 x 18 cm) which had been equilibrated with the buffer solution containing 5mM dithiothreitol for affinity chromatography. The column was washed with 150 ml of the same buffer solution and then was eluted with the same buffer solution containing 5mM NAD$^+$. Each 1 ml fraction was collected. Secondary alcohol dehydrogenase activity was located in tube nos. 10-15.

A summary of the purification steps and the properties of the fraction obtained from each is given in Table IV. The activity of the NAD$^+$-linked secondary alcohol dehydrogenase in each fraction was measured using a fluorescence spectrophotometer by monitoring the formation of reduced NAD$^+$ (excitation at 340 nm, emission at 460 nm). The assay system (3 ml) contained 150 $\mu$ mole sodium phosphate buffer pH 7.0, 1.0 $\mu$ mole NAD$^+$, a given amount of enzyme fraction, and 20 $\mu$ mole 2-propanol substrate. One unit of enzyme activity represents the reduction of 1 nmole of NAD$^+$ per minute. Protein concentrations in each fraction were determined by the method of O.H. Lowry et al., J. Biol. Chem., 193, 255 (1951).

TABLE IV

Separation and Purification of Alcohol Dehydrogenase Enzymes
from Propane-Grown Pseudomonas fluorescens NRRL B-1244

| Step | Protein (mg) | Total Activity (units) | Specific Activity (units/mg) | Yield (%) | Purification (fold) |
|---|---|---|---|---|---|
| 1. crude extract | 12550 | 376,500 | 30 | 100 | 1 |
| 2. fractionation with ammonium sulfate | 4280 | 359,520 | 84 | 95 | 2.8 |
| 3. DEAE-cellulose column chromatography | 966 | 239,568 | 248 | 63 | 8.2 |
| 4. Polyacrylamide gel column chromatography (best fraction) | 126 | 108,486 | 861 | 28 | 28.7 |
| 5. Agarose Affinity chromatography | 30 | 42,000 | 1,400 | 11 | 46 |

EXAMPLE 3

Properties of Purified Secondary Alcohol Dehydrogenase Enzyme
Purity and Molecular Weight

The purified enzyme fraction obtained from step 5 of Example 2 exhibited a single protein band in polyacrylamide gel electrophoresis, conducted in a 7.5% gel and using stains with both Coomassie brillant blue as described by S. Raymond, Clin. Chem., 8, 455 (1962), and with nitro-blue tetrazolium activity stain as described by G.J. Brewer, An Introduction to Isozyme Techniques (Academic Press: New York, 1970) pp. 117-119. The molecular weight of the secondary alcohol dehydrogenase determined by a calibrated polyacrylamide gel column chromatography analysis was 145,000 dalton. Electrophoresis in polyacrylamide gel in the presence of sodium dodecyl sulfate (0.1%) gave a single protein component with a molecular weight of 40,000 dalton, indicating that secondary alcohol dehydrogenase consists of four subunits of identical molecular weight (40,000 dalton per molecule of subunit protein).

Substrate Specificity

The purified enzyme from step 5 of Example 2 was used in the procedure of Example 1 to oxidize primary and secondary alcohols, diols and aldehydes. The specificity of the dehydrogenase for the substrates is expressed as nmoles of product formed per min. per 85 µg of protein and as a percentage relative to the oxidation rate of 2-propanol (designated as 100% oxidation rate) in Table V.

## Table V

Relative Reaction Rate of Secondary Alcohol Dehydrogenase from Propane-Grown <u>Pseudomonas fluorescens</u> NRRL B-1244 for Various Substrates

| Substrates | Rate of Oxidation | |
|---|---|---|
| | (nmoles/min./ 85 ug μg protein) | (percent relative to 2-propanol) |
| Secondary Alcohols: | | |
| 2-propanol | 119 | 100 |
| 2-butanol | 206 | 170 |
| 2-pentanol | 80 | 67 |
| 3-pentanol | 60 | 50 |
| 2-hexanol | 0 | 0 |
| 2-heptanol | 0 | 0 |
| Primary alcohols: | | |
| methanol | 6 | 5 |
| ethanol | 15 | 12 |
| 1-propanol | 34 | 28 |
| 1-butanol | 26 | 21 |
| 1-pentanol | 40 | 33 |
| 1-hexanol | 0 | 0 |
| Aldehydes: | | |
| formaldehyde | 7 | 6 |
| propanal | 36 | 30 |
| butanal | 15 | 12 |
| pentanal | 0 | 0 |
| hexanal | 0 | 0 |
| decanal | 0 | 0 |
| benzaldehyde | 0 | 0 |
| Diols: | | |
| 1,3-butanediol | 116 | 97 |
| 1,2-butanediol | 49 | 41 |
| 2,3-butanediol | 39 | 32 |
| 1,2-propanediol | 60 | 50 |
| Branched alcohols: | | |
| isobutanol | 68 | 12 |
| isopentyl alcohol | 0 | 0 |
| Cyclic and aromatic alcohols: | | |
| benzyl alcohol | 2.4 | 2 |
| cyclohexanol | 25 | 21 |
| Stereospecificity: | | |
| (+)-2-butanol | 206 | 170 |
| (-)-2-butanol | 206 | 170 |

The results indicate that the secondary alcohol dehydrogenase enzyme has a broad substrate specificity, being active on short-chain 2-alcohols, aldehydes, 1-alcohols and certain diols, with the highest oxidation rate for 2-butanol. Branched-chain alcohols and cyclic and aromatic alcohols were also oxidized, at a lower rate. The enzyme showed no preference for the stereospecificity of the substrates as indicated by the equal oxidation rates of the (+) - and (-) isomers of 2-butanol.

When $NADP^+$ was used as a cofactor for the secondary alcohol dehydrogenase, the oxidation rate was about 50% that for $NAD^+$. The reduction of acetone to propanol by this enzyme in the presence of $NADH_2$ (the reverse reaction) occurred, but the oxidation rate was about 20% of that observed for the oxidation (forward reaction).

## Effect of pH

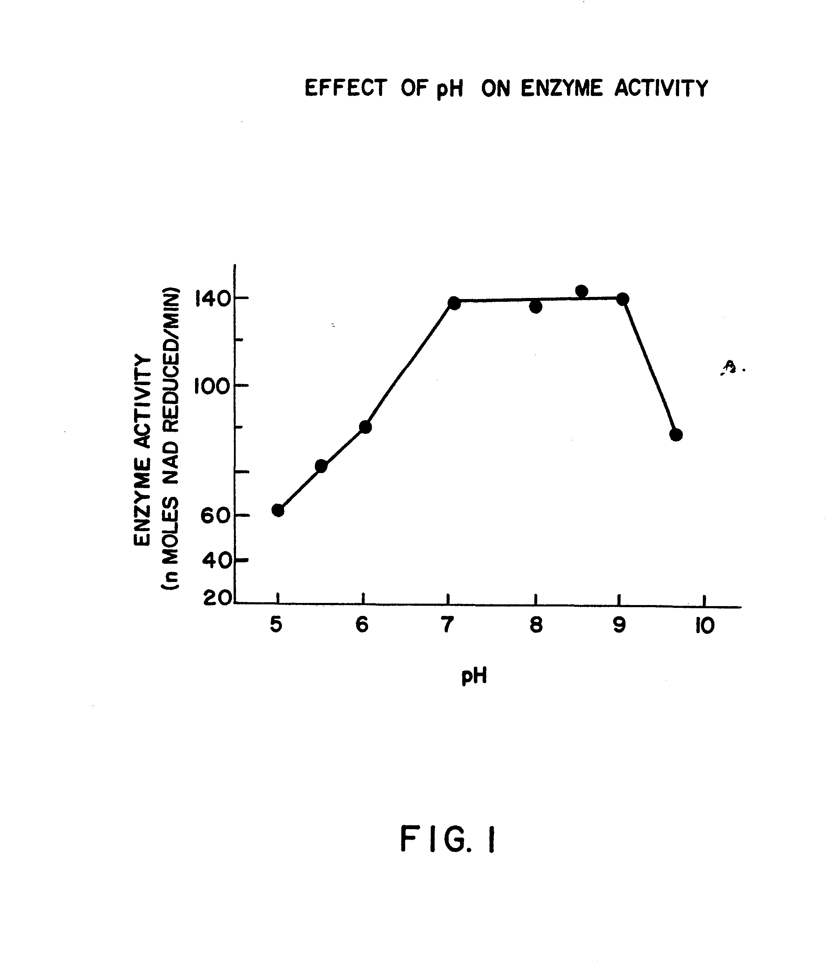

The effect of pH on the secondary alcohol dehydrogenase enzyme activity was studied in the pH range of 5-10 using 0.05 M buffer solutions (sodium phosphate buffer for pH 5-8; tris-HCl buffer for pH 8-10). Enzyme activity was measured at 25°C according to the method described in Example 2 using 5 µgrams enzyme. A plot of enzyme activity versus pH is provided in Figure 1. It can be seen that the optimum pH for enzyme activity was found to be about 7-9, where the activity reached almost 140 nmoles NAD reduced per minute.

## Effect of Temperature

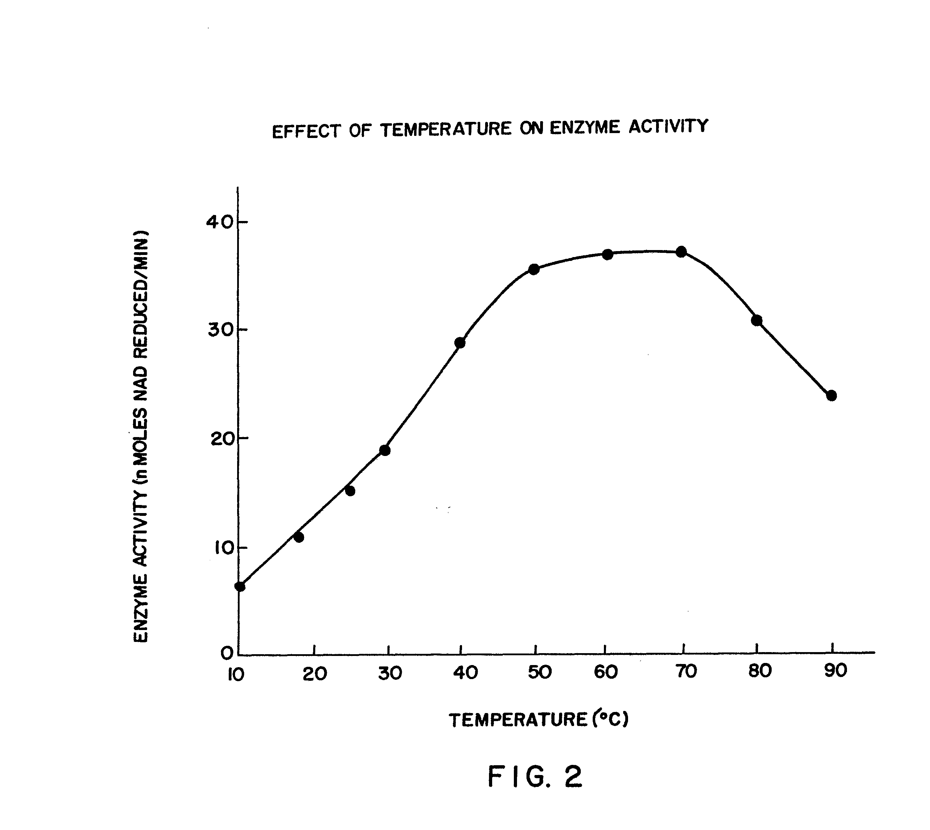

The effect of temperature on secondary alcohol dehydrogenase enzyme activity was determined in phosphate buffer pH 7.0 because the pH of this buffer is not drastically altered by changes in temperature. The reaction mixture was preincubated, in the presence of enzyme (1.5 μg), at from 10 to 90°C for 5 minutes, and then the reaction was initiated by the addition of 2-propanol substrate at the same temperature. A plot of enzyme activity versus temperature is provided in Figure 2. The results indicate that the optimum temperature for the dehydrogenase-catalyzed reaction was 60-70°C, where the activity was about 34-38 nmoles $NAD^+$ reduced per minute, but the activity was still fairly high (about 22-23 nmoles $NAD^+$ reduced per minute) even at a temperature of 90°C. The activation energy for secondary alcohol dehydrogenase, as calculated from an Arrhenius-plot of velocity vs. the reciprocal of the absolute temperature, is 8.2 kcal.

## Thermal Stability of Enzyme

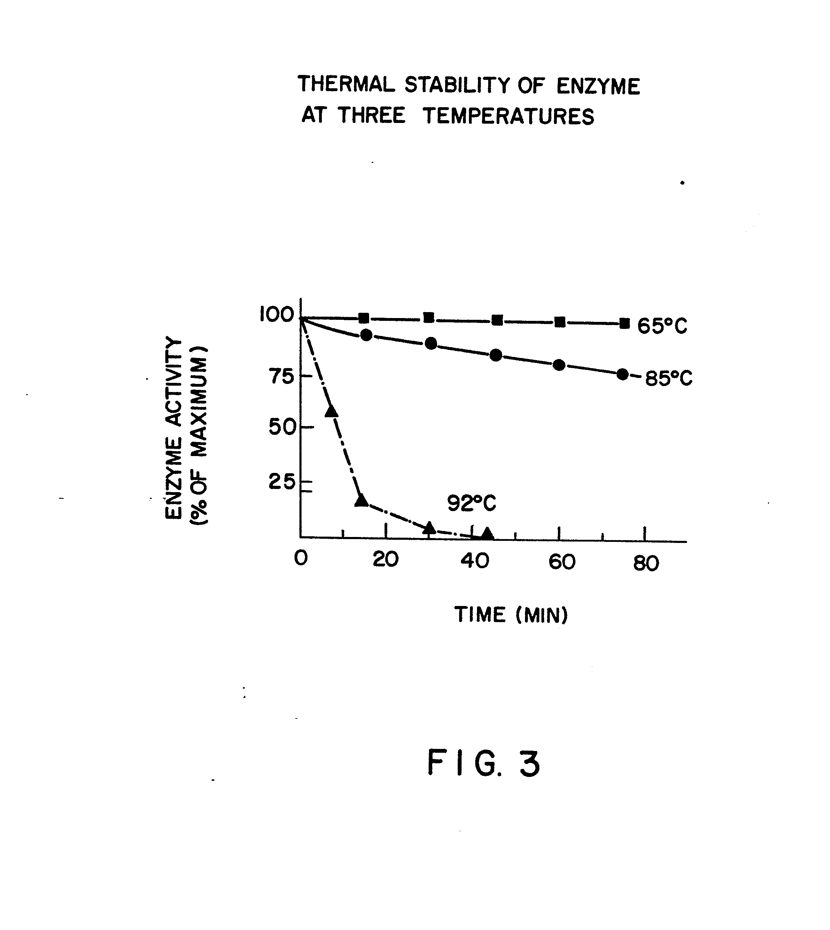

The thermal stability of the NAD-linked secondary alcohol dehydrogenase enzyme was also investigated using dilute enzyme solution (300 μg/ml) in the presence of air and without adding a thiol protecting agent. A plot of thermal stability (enzyme activity) versus time at three different temperatures is provided in Figure 3. It is seen that enzyme activity was not influenced by heating at 65°C for 75 min. The enzyme displayed good stability toward heating at 85°C for 75 min. but was deactivated sharply at 92°C and was denatured on boiling. Enzyme thermal stability and activity were not altered by enzyme freezing and thawing.

## Michaelis Constants

The effect of $NAD^+$ and 2-propanol concentrations on the enzyme activity was studied at 25°C in 0.05 M sodium phosphate buffer pH 7.0. 2-Propanol was kept at a saturated level (6.6 mM) for the determination of the $K_m$ value for $NAD^+$; and $NAD^+$ was maintained at a saturated level (1mM) for determining the Km value for

2-propanol. The $K_m$ values calculated from Lineweaver-Burk plots were 8.2 x $10^{-6}$ M for $NAD^+$ and 8.5 x $10^{-5}$ M for 2-propanol. It is noted that the lower the $K_m$ value the more active is the enzyme.

Effect of Potential Inhibitors

The oxidation of 2-propanol by the purified extract of secondary alcohol dehydrogenase enzyme was conducted in the presence of a 1mM concentration of one of the metal chelating agents and thio-reagents listed in Table VI.

TABLE VI

Inhibition of Secondary Alcohol Dehydrogenase Activity

| Inhibitors | % Inhibition |
|---|---|
| thiosemicarbazide | 44 |
| iodoacetic acid | 0 |
| acetamide | 0 |
| imidazole | 0 |
| N-ethylmaleimide | 33 |
| 5,5'-dithiobis-(2-nitrobenzoate) | 100 |
| p-hydroxymercuribenzoate | 100 |
| ethylenediamine tetraacetic acid (EDTA) | 0 |
| potassium cyanide | 0 |
| 1,10-phenanthroline | 100 |
| $\alpha$,$\alpha$'-dipyridyl | 40 |
| thiourea | 0 |
| 5-nitro-8-hydroxyquinoline | 100 |
| $Cu^{2+}$ | 10 |
| $Hg^{2+}$ | 100 |

It can be seen that the activity of the enzyme was inhibited by strong thio-reagents such as p-hydroxy-mercuribenzoate, 5,5'-dithiobis-(2-nitrobenzoic acid), and 5-nitro-8-hydroxyquinoline. The metal chelating agents, except 1,10-phenanthroline and the mercuric ion, did not inhibit the enzyme activity.

Effect of 2-Butanol

The activity of secondary alcohol dehydrogenase was not inhibited by the presence of up to 400 mM 2-butanol in the oxidation reaction mixture. The enzyme still demonstrated 40% of its original activity in a 0.9 M 2-butanol solution and 30% of its activity in a 1.1 M 2-butanol solution.

In summary, the present invention is seen to provide a thermally stable alcohol dehydrogenase enzyme with a broad substrate specificity preferential to secondary alcohols but also converting primary alcohols, diols, aldehydes and methyl ketones to their respective conversion products, and a thermally labile alcohol dehydrogenase enzyme preferential to primary alcohols.

CLAIMS:

1. A primary or secondary alcohol dehydrogenase enzyme which is derived from cells of a microorganism or genetically engineered derivative thereof or natural mutant thereof which has been previously grown under aerobic conditions in a nutrient medium containing a carbon-containing compound having at least two carbon atoms which provides the carbon and energy source for growth of the cells of the microorganism and induces alcohol dehydrogenase enzyme activity in the microorganism, derivative or mutant.

2. A primary alcohol dehydrogenase enzyme according to claim 1 characterized as converting primary alcohols to the corresponding oxidation products under aerobic conditions in the presence of $NAD^+$ or $NADP^+$.

3. A secondary alcohol dehydrogenase enzyme according to claim 1 characterized as converting short-chain primary and secondary alcohols, diols and aldehydes to the corresponding oxidation products in the presence of $NAD^+$ or $NADP^+$.

4. A secondary alcohol dehydrogenase enzyme which has a molecular weight of about 145,000 ± 5,000 dalton as determined by polyacrylamide gel column chromatography and is stable at a temperature of at least 50°C for over one hour.

5. A dehydrogenase enzyme according to claim 4 which is stable at 60-85°C for over one hour.

6. A process for the microbiological conversion of alcohols, diols and aldehydes to the corresponding oxidation products, or of methyl ketones to alcohols, which comprises contacting said alcohol, diol, aldehyde or ketone in a reaction mixture with microbial cells derived from a microorganism, a genetically engineered derivative or natural mutant thereof, or an enzyme preparation prepared from said cells, which cells have been previously heated at a temperature of up to about 90°C and which microorganism has been previously grown under aerobic conditions in a nutrient medium containing a carbon-containing compound having at least two carbon atoms which provides the carbon and energy source for growth of the cells of the microorganism and induces alcohol dehydrogenase enzyme activity in the microorganism, derivative, mutant or preparation, and producing the product in isolatable amounts.

7. A process according to claim 6 in which the alcohol is a $C_1$-$C_5$ primary alcohol or $C_3$-$C_7$ secondary alcohol, the diol is a $C_3$-$C_5$ diol, the aldehyde is a $C_1$-$C_4$ aldehyde, and the methyl ketone is acetone.

8. A process according to claim 6 or 7 in which the microorganism is bacteria, fungi or yeast grown under aerobic conditions in the presence of a $C_2$-$C_{10}$ alkyl compound.

9. A process according to any one of claims 6-8 in which the enzyme preparation is cell-free and the reaction medium additionally contains nicotinamide adenine dinucleotide.

anyone of
10. A process according to /claims 6-9 in which the enzyme preparation is substantially purified secondary alcohol dehydrogenase.

EFFECT OF pH ON ENZYME ACTIVITY

FIG. I

EFFECT OF TEMPERATURE ON ENZYME ACTIVITY

FIG. 2

THERMAL STABILITY OF ENZYME
AT THREE TEMPERATURES

FIG. 3